Europäisches Patentamt

European Patent Office

Office européen des brevets

(11) Publication number: **0 198 476 B1**

# EUROPEAN PATENT SPECIFICATION

(45) Date of publication of patent specification: **07.08.91**   (51) Int. Cl.⁵: **C07C 37/07**

(21) Application number: **86105190.2**

(22) Date of filing: **15.04.86**

(54) Process for the preparation of 2,3,5.trimethyl-hydroquinone.

(30) Priority: **16.04.85 JP 80948/85**

(43) Date of publication of application:
**22.10.86 Bulletin 86/43**

(45) Publication of the grant of the patent:
**07.08.91 Bulletin 91/32**

(84) Designated Contracting States:
**CH FR LI**

(56) References cited:
**US-A- 3 197 398**
**US-A- 3 236 903**
**US-A- 4 247 720**

(73) Proprietor: **MITSUBISHI GAS CHEMICAL COMPANY, INC.**
**5-2, Marunouchi 2-chome Chiyoda-Ku**
**Tokyo, 100(JP)**

(72) Inventor: **Yui, Tomoyuki**
**22-1, 3-chome Miyazono**
**Nagareyama-shi Chiba-ken(JP)**
Inventor: **Ito, Akira**
**18-7, 7-chome Matsuba-cho**
**Kashiwa-shi Chiba-ken(JP)**
Inventor: **Takata, Toshiaki**
**11-16, 5-chome Kana-machi**
**Katsushika-ku Tokyo(JP)**

(74) Representative: **Türk, Dietmar, Dr. rer. nat. et al**
**Türk, Gille + Hrabal Patentanwälte Brucknerstrasse 20**
**W-4000 Düsseldorf 13(DE)**

**Description**

The present invention relates to a process for the preparation of 2,3,5-trimethylhydroquinone (hereinafter referred to as TMHQ). More particularly it is concerned with a process for preparing TMHQ which comprises reducing 2,3,5-trimethylbenzoquinone (hereinafter referred to as TMBQ) with hydrogen in the presence of a catalyst comprising a platinum group metal deposited on zeolite as a carrier.

TMHQ is important as one of the starting materials for the preparation of vitamin E (α-tocopherol). In recent years, therefore, TMHQ has been increasingly demanded. The quality of vitamin E varies with the purity of TMHQ as a starting material. Thus, in the preparation of vitamin E, it is required to use TMHQ of high purity from an economic standpoint and also from a standpoint of product quality.

It has been known that there are various methods of preparation of TMHQ which use varied starting materials. Of these methods, a method of preparing TMHQ through TMBQ is most promising.

For the preparation of TMHQ using TMBQ as a starting material, there is known a method of reducing TMBQ with a reductant such as sodium hydrogensulfite (see Japanese Patent Application Laid-Open No. 108032/1974) and sulfur dioxide (see Japanese Patent Publication No. 14455/1984). These methods, however, have disadvantages in that the reductant is expensive, the yield is low, and impurities derived from the reductant are readily intermingled with the product.

In addition, a method in which TMBQ is reduced with hydrogen in the presence of a catalyst is known (see, for example, U.S. Patent 2,229,573). This method has advantages in that the reductant is inexpensive and furthermore is easy to separate because it is gas. Catalysts comprising an activated metal such as Raney nickel and Raney cobalt, rhenium or a platinum group metal such as palladium, platinum, ruthenium and rhodium supported on a carrier are known to be able to use in the above method (see Japanese Patent Publication No. 20285/1982). These catalysts can be easily separated from TMHQ and the solvent by filtration after the reaction is completed, because they are solid. As described above, this hydrogen reduction process has various advantages.

TMHQ is required to have a purity as high as not less than 99% since it is used as a starting material for the preparation of vitamin E which is to be utilized as a medicine of food additive. If the purity of TMHQ is lower than the above value, it is necessary to be purified. For this purification, several methods have been proposed. One of the methods is to crystallize TMHQ out of the reaction mixture by adding a poor solvent after hydrogen reduction is completed (see Japanese Patent Publication Nos. 26424/1976 and 20285/1982). Another method is to recrystallize TMHQ once separated from the reaction mixture from a specific mixed solvent (see Japanese Patent Publication No. 20285/1982). These purification methods, however, have disadvantages in that a large amount of a solvent should be used, solvents constituting the mixed solvent should be separated and recovered, and in that if the purity of TMHQ is low, the energy load is increased. Furthermore, the amount of TMHQ discarded together with impurities is increased with a reduction in the purity of TMHQ, leading to a decrease in yield.

In a case where the purity of TMHQ is sufficiently high, the desired product can be obtained without any purification and even if purification is applied, the energy load needed for the purification can be greatly decreased.

Thus the selectivity in hydrogen reduction of TMBQ is a very important factor which influences on the yield and purity of TMHQ and production costs of TMHQ. It has therefore been desired to establish a method whereby hydrogen reduction can be attained in a high selectivity.

In industrial application of catalysts, the service life of the catalyst is important from a standpoint of convenience of operation and also from an economic standpoint. That is, it is essential for industrially used catalysts that not only activity and selectivity at an earlier stage of use are high but also such high activity and selectivity are maintained even when they are used for a long time. Also as for catalysts for hydrogen reduction of TMBQ, the service life is an important factor.

As carriers for platinum group metals to be used in hydrogen reduction of TMBQ, active carbon (U.S. Patent 2,229, 573), alumina (Japanese Patent Application Laid-Open No. 100430/1977), silica, pumice, silicic acid (Japanese Patent Publication No. 23487/1976), and the like have heretofore been known. However, in hydrogenation of TMBQ with catalysts comprising platinum group metals deposited on the above carriers, side reactions such as addition of hydrogen to a carbon-carbon double bond occur, resulting in a decrease of selectivity to the desired TMHQ. In order to obtain a high purity product, it is necessary that the reaction mixture be highly purified.

These catalysts, moreover, cannot be said to have a sufficiently long service life. Thus several attempts have been made to lengthen the service life (see, for example, Japanese Patent Publication No. 42964/1979) but with no satisfactory results.

An object of the present invention is to provide a process for the preparation of high purity TMHQ through hydrogen reduction of TMBQ.

Another object of the present invention is to provide a method of hydrogen reduction of TMBQ using a platinum group metal catalyst deposited on zeolite whereby TMHQ can be obtained with high selectivity.

Still another object of the present invention is to provide a platinum group metal catalyst for hydrogen reduction of TMBQ, which can be used for a long period of time with high activity and high selectivity.

As a result of extensive investigations, it has been found that the above objects can be attained by reducing TMBQ with hydrogen in the presence of a platinum group metal catalyst prepared using zeolite as a carrier.

The present invention relates to a process for preparing TMHQ by reducing TMBQ with hydrogen in the presence of a platinum group metal catalyst wherein the catalyst comprises zeolite as a carrier and a platinum group metal deposited on the zeolite.

In accordance with the process of the present invention, TMBQ can be hydrogenated to TMHQ with high selectivity and, therefore, high purity TMHQ can be easily prepared without any post-treatment.

Furthermore, since the service life of the catalyst is long, it is not necessary for the catalyst to be changed frequently and thus the operation can be carried out economically.

Zeolite which is used as a carrier in the present invention is aluminosilicic acid having a three-dimensional framework, or its alkali or alkaline earth metal salt. Both natural and synthetic zeolites can be used.

According to type of framework, zeolites used in the present invention can be classified as follows;

(i) Natrolite group (natrolites) such as natrolite, mesolite, thomsonite, gonnardite, scolecite, and edingtonite

(ii) Mordenite group (mordenites) such as mordenite and dachiardite

(iii) Faujasite group (faujasites) such as foujasite

(iv) Chabazite group (chabazites) such as chabazite and gmelinite

(v) Analcime group (analcines) such as analcime, wairakite and leucite

(vi) Phillipsite group (phillipsites) such as phillipsite, harmotome, and gismondine

(vii) Structurally non-classified group such as clinoptilolite, erionite, heulandite, stilbite, epistilbite, brewsterite, ferrierite, laumontite, levyne, and ashcroftine

(viii) Synthetic zeolites of the type other than the above groups, such as A type molecular sieve

In the process of the present invention, zeolites classified in the above (i) to (viii) can be used singly or in combination with each other. Of these compounds, zeolites belonging to (ii) mordenite group, (iii) faujasite group, (iv) chabazite group, (vii) clinoptilolite, erionite and (viii) A type molecular sieve are preferred.

Platinum group metals which can be used are palladium, platinum, ruthenium, rhodium, iridium, and osmium. Of these metals, palladium, platinum, ruthenium and rhodium are preferred. More preferred are palladium and platinum, and most preferred is palladium.

The metal component can be deposited on the zeolite by the usual method which permits deposition of platinum group metals and their compounds. Preferred are an ion exchange method and an impregnation method.

The metal component to be deposited is in a soluble form, such as chloride, ammine complex salt, acetate and acetyl acetonate, preferably in the form of chloride or ammine complex salt. Most preferred is the ammine complex salt.

The platinum group metal component can be reduced to the metal by the usual method using reductants. For example, reductants such as hydrogen, formalin, sodium formate, carbon monoxide and hydrazine can be used. Preferred is a hydrogen reduction method.

The amount of the platinum group metal deposited on the carrier is usually 0.1 to 10 wt%, preferably 0.3 to 2 wt% based on the weight of the carrier.

The amount of the catalyst comprising platinum group metal and carrier is usually 0.5 to 30 wt%, preferably 1 to 10 wt% based on the weight of TMBQ.

As solvents, those capable of dissolving TMBQ, TMHQ and their adduct, i.e., quinhydrone can be used. Solvents which can be used include aliphatic alcohols such as propyl alcohol, butyl alcohol and amyl alcohol as well as methanol and ethanol which have been believed unsuitable because they are responsible for coloration, aromatic alcohols such as benzyl alcohol, ketones such as acetone and methyl ethyl ketone, organic acids such as acetic acid and propionic acid, organic acid esters such as methyl propionate, ethyl acetate, propyl acetate and butyl formate, ethers such as dipropyl ether and dibutyl ether, cyclic ethers such as tetrahydrofuran and dioxane, and lactones such as $\gamma$-butyrolactone. These solvents can be used in combination with each other. Of these solvents, alcohols, ketones, organic acid esters and ethers are preferred. From standpoints of solubility and ease of separation and also an economic standpoint, alcohols are more preferred. Most preferred are methanol and ethanol.

3

Hydrogen to be used for reducing TMBQ is not necessary to be pure. If necessary, mixed gases of hydrogen and inert gases such as nitrogen and carbon dioxide gas can be used.

The hydrogen partial pressure is usually 0.01 to 20 $kg/cm^2$ and preferably 0.1 to 10 $kg/cm^2$.

The suitable reaction temperature is usually 10 to 100°C, preferably 20 to 60°C although it varies depending on the vapor pressure of the solvent to be used.

It is preferred for the solvent to be used in a sufficient amount to dissolve the starting material of TMBQ, the product of TMHQ and an adduct of TMBQ and TMHQ of quinhydrone. Although the amount of the solvent used varies depending on the type of the solvent, the solvent is usually used in such an amount that the concentration of the TMBQ starting material is 5 to 30 wt%.

In accordance with the process of the present invention, TMHQ of purity as high as not less than 99% can be easily prepared by hydrogen reduction of TMBQ. Thus the present invention has a great advantage in that high purity TMHQ can be easily prepared without any post-treatment.

Furthermore, since the catalyst of the present invention has a long service life, the operation can be carried out economically.

The present invention is described below in greater detail with reference to the following examples.

### EXAMPLE 1

#### Preparation of Catalyst

An aqueous solution of $Pd(NH_3)_4Cl_2$ was added to Zeolon 900H (mordenite, synthetic product, produced by Norton Co., Ltd.), which had been ion exchanged to the Na type, to thereby achieve ion exchange. The resulting mixture was filtered, and the residue was calcined in air at 400°C and reduced with hydrogen to prepare a catalyst. This catalyst consists of the Zeolon and 1% Pd deposited thereon. This is hereinafter referred to as "Preparation Method 1".

#### Hydrogen Reduction

A mixture of 1 g of 1% Pd-deposited Zeolon 900H (1% Pd/Zeolon 900H) as prepared above, 15 g of TMBQ and 45 g of methanol was placed in a reactor and the atmosphere in the reactor was replaced with hydrogen. Then the mixture was stirred in a hydrogen atmosphere at 40°C and ordinary pressure while compensating for consumed hydrogen until the consumption of hydrogen stopped. It took 46 minutes. After the reaction was completed, the catalyst was filtered off and methanol was distilled away from the resulting filtrate. On drying the resulting residue, white crystals of TMHQ were obtained. The conversion was 100% and the selectivity was 99.9% as determined by gas chromatography.

### EXAMPLE 2

TMHQ was prepared in the same manner as in Example 1 except that Zeaklite (clinoptilolite, natural product, produced by zeaklite Chemical Co., Ltd.) was used in place of Zeolon 900H. The selectivity was 99.9%.

### EXAMPLE 3

TMHQ was prepared in the same manner as in Example 1 except that Zeolon 500 (a mixture of chabazite and erionite, natural product, produced by Norton Co., Ltd.) was used in place of Zeolon 900H. The selectivity was 99.8%.

### EXAMPLE 4

TMHQ was prepared in the same manner as in Example 1 except that Molecular Sieve 13X (faujasite, synthetic product, produced by Gasukuro Kogyo Inc.) was used in place of Zeolon 900H. The selectivity was 99.8%.

### EXAMPLE 5

TMHQ was prepared in the same manner as in Example 1 except that Molecular Sieve 4A (no corresponding natural product exists; synthetic product, produced by Gasukuro Kogyo Inc.) was used in place of Zeolon 900H. The selectivity was 99.8%.

EXAMPLE 6

Preparation of Catalyst

Zeolon 900H was impregnated with an aqueous solution of $Pd(NH_3)_4Cl_2$. Water was removed by evaporation, and the residue was calcined in air at 400°C and reduced with hydrogen to prepare a catalyst. This catalyst consists of the Zeolon and 1% Pd deposited thereon. This is hereinafter referred to as Preparation Method 2.

Hydrogen Reduction

On repeating the same procedure as in Example 1 except that the above prepared 1% Pd/Zeolon 900H was used, white crystals of TMHQ were obtained. The selectivity was 99.8%.

EXAMPLE 7

TMHQ was prepared in the same manner as in Example 6 except that Zeaklite was used in place of Zeolon 900H and the reaction time was 39 minutes. The selectivity was 99.9%.

EXAMPLE 8

Preparation of Catalyst

Zeolon 900H was impregnated with an aqueous acidic solution with hydrochloric acid of $PdCl_2$. Water was removed by evaporation, and the residue was calcined in air at 400°C and then reduced to prepare a catalyst. This catalyst consists of the Zeolon and 1% Pd deposited thereon This is hereinafter referred to as Preparation Method 3.

Hydrogen Reduction

TMHQ was prepared in the same manner as in Example 1 except that the above prepared 1% Pd/Zeolon 900H was used in place of 1% Pd/Zeolon 900H of Example 1. The selectivity was 99.5%.

EXAMPLE 9

TMHQ was prepared in the same manner as in Example 8 except that Zeaklite was used in place of Zeolon 900H. The selectivity was 99.6%.

EXAMPLE 10

TMHQ was prepared in the same manner as in Example 1 except that a mixed gas atmosphere of hydrogen and nitrogen (1:1 by volume) was used in place of the hydrogen atmosphere. The selectivity was 99.9%.

EXAMPLE 11

TMHQ was prepared in the same manner as in Example 1 except that 0.5 g of 2% Pd/Zeolon 900H which had been prepared in accordance with Preparation Method 1 was used in place of 1 g of 1% Pd/Zeolon 900H, the reaction temperature was 20°C and the amount of the methanol used was 60 g. The selectivity was 99.8%.

EXAMPLE 12

TMHQ was prepared in the same manner as in Example 7 that the reaction temperature was 60°C and 60 g of ethanol was used in place of methanol. The selectivity was 99.8%.

EXAMPLE 13

TMHQ was prepared in the same manner as in Example 7 except that the reaction temperature was 60° C and the gauge pressure was 2 kg/cm². The selectivity was 99.8%.

EXAMPLE 14

TMHQ was prepared in the same manner as in Example 8 except that 1% Pt/Zeolon 900H which had been prepared in accordance with Preparation Method 3 using $H_2PtCl_6$ in place of $PdCl_2$ was used in place of 1% Pd/Zeolon 900H. The selectivity was 99.4%.

EXAMPLE 15

TMHQ was prepared in the same manner as in Example 14 except that 1% Rh/Zeolon 900H which had been prepared in accordance with Preparation Method 3 using $RhCl_3$ in place of $PdCl_2$ was used in place of 1% Pt/Zeolon 900H. The selectivity was 99.3%.

EXAMPLE 16

TMHQ was prepared in the same manner as in Example 14 except that 1% Ru/Zeolon 900H which had been prepared in accordance with Preparation Method 3 using $RuCl_3$ in place of $PdCl_3$ was used in place of 1% Pt/Zeolon 900H. The selectivity was 99.3%.

EXAMPLE 17

The reaction was repeated in the same manner as in Example 1 except for using the catalyst which had been separated by filtration after completion of the reaction in Example 1. The time (reaction time) until consumption of hydrogen stopped was gradually lengthened as follows.

|  | Reaction time (min.) |
|---|---|
| 1st batch (Example 1) | 46 |
| 20th batch | 53 |
| 40th batch | 64 |
| 60th batch | 81 |
| 71st batch | 92 |
| 80th batch | 107 |
| 88th batch | 120 |

The selectivity of TMHQ at the 88th batch was 99.8%.

COMPARATIVE EXAMPLE 1

TMHQ was prepared in the same manner as in Example 1 except that 1% Pd/Al₂O₃ (produced by Nippon Engelhard Ltd.) was used in place of 1% Pd/Zeolon 900H, and the reaction time (the time until consumption of hydrogen stopped) was 24 minutes. The selectivity was 97.7%.

COMPARATIVE EXAMPLE 2

TMHQ was prepared in the same manner as in Example 1 except that 1% Pd/active carbon (produced by Nippon Engelhard Ltd.) was used in place of 1% Pd/Zeolon 900H, and the reaction time was 20 minutes. The selectivity was 98.2%.

COMPARATIVE EXAMPLE 3

The reaction was repeated using 1% Pd/Al₂O₃ used in Comparative Example 1. The reaction time was

6

lengthened as follows.

|  | Reaction time (min.) |
|---|---|
| 1st batch (Comparative Example 1) | 24 |
| 10th batch | 35 |
| 16th batch | 48 |
| 20th batch | 64 |
| 26th batch | 126 |

COMPARATIVE EXAMPLE 4

The reaction was repeated using 1% Pd/active carbon used in Comparative Example 2. The reaction time was lengthened as follows.

|  | Reaction time (min.) |
|---|---|
| 1st batch (Comparative Example 2) | 20 |
| 10th batch | 33 |
| 13th batch | 40 |
| 20th batch | 83 |
| 22nd batch | 125 |

COMPARATIVE EXAMPLE 5

The same procedure was carried out as in Comparative Example 3 except that 1% Pd/SiO$_2$·Al$_2$O$_3$ - (produced by Nippon Engelhard Ltd.) was used in place of 1% Pd/Al$_2$O$_3$. The reaction time was lengthened as follows.

|  | Reaction time (min.) |
|---|---|
| 1st batch | 25 |
| 10th batch | 36 |
| 16th batch | 50 |
| 20th batch | 67 |
| 26th batch | 139 |

**Claims**

1. In a process for preparing 2,3,5-trimethylhydroquinone by reducing 2,3,5-trimethylbenzoquinone with hydrogen in a solvent in the presence of a catalyst, the improvement which comprises using, as the catalyst, platinum group metal deposited on zeolite.

2. A process as claimed in Claim 1, wherein the zeolite is at least one material selected from the group consisting of mordenites, faujasites, chabazites, A type molecular sieve, clinoptilolite, and erionite.

3. A process as claimed in Claim 1, wherein the platinum group metal is at least one metal selected from the group consisting of palladium, platinum, ruthenium, and rhodium.

4. A process as claimed in Claim 1, wherein the platinum group metal is palladium, platinum, or mixture thereof.

5. A process as claimed in Claim 1, wherein the platinum group metal is palladium.

6. A process as claimed in Claim 1, wherein the catalyst is a material obtained by depositing platinum group metal compound on zeolite and then reducing the compound.

7. A process as claimed in Claim 6, wherein the deposition of platinum group metal compound on zeolite is carried out by an ion-exchange method or an impregnation method.

8. A process as claimed in Claim 6, wherein the platinum group metal compound is chloride or ammine complex salt.

9. A process as claimed in Claim 6, wherein the reduction is carried out with hydrogen.

10. A process as claimed in Claim 1, wherein the amount of the platinum group metal deposited is from 0.1 to 10 percent by weight based on the zeolite.

11. A process as claimed in Claim 1, wherein the amount of the platinum group metal deposited is from 0.3 to 2 percent by weight based on the zeolite.

12. A process as claimed in Claim 1, wherein the amount of the catalyst is from 0.5 to 30 percent by weight based on the 2,3,5-trimethylbenzoquinone.

13. A process as claimed in Claim 1, wherein the solvent is an aliphatic alcohol.

14. A process as claimed in Claim 1, wherein the solvent is methanol or ethanol.

15. A process as claimed in Claim 1, wherein the reduction of 2,3,5-trimethylbenzoquinone with hydrogen is carried out under a hydrogen partial pressure of from 0.01 to 20 kg/cm$^2$.

16. A process as claimed in Claim 1, wherein the reduction of 2,3,5-trimethylbenzoquinone with hydrogen is carried out at a temperature of from 10 to 100$^\circ$C.

**Revendications**

1. Perfectionnement qui consiste à utiliser comme catalyseur un métal du groupe platine déposé sur zéolite, dans un procédé de préparation au 2,3,5-triméthylhydroquinone en réduisant le 2,3,5-triméthylbenzoquinone par l'hydrogène dans un solvant en présence d'un catalyseur.

2. Procédé selon la revendication 1, dans lequel le zéolite est au moins un matériau choisi dans le groupe comprenant les mordenites, les faujasites, les chabazites, les tamis moléculaires de type A, la clinoptilolite et l'érionite.

3. Procédé selon la revendication 1, dans lequel le métal du groupe platine est au moins un métal choisi dans le groupe comprenant le palladium, le platine, le ruthénium et le rhodium.

4. Procédé selon la revendication 1, dans lequel le métal du groupe platine est le palladium, le platine ou le mélange des deux.

5. Procédé selon la revendication 1, dans lequel le métal du groupe platine est le palladium.

6. Procédé selon la revendication 1, dans lequel le catalyseur est un matériau obtenu en déposant un composé de métal du groupe platine sur de la zéolite puis en réduisant le composé.

7. Procédé selon la revendication 6, dans lequel le dépôt du composé métal du groupe platine sur la zéolite s'effectue par une méthode d'échange d'ions ou d'imprégnation.

8. Procédé selon la revendication 6, dans lequel le composé métallique du groupe platine est un chlorure ou un sel complexe ammine.

9. Procédé selon la revendication 6, dans lequel la réduction s'effectue par de l'hydrogène.

10. Procédé selon la revendication 1, dans lequel la quantité de métal du groupe platine est déposée à raison de 0,1 à 10 pour cent du poids du zéolite.

11. Procédé selon la revendication 1, dans lequel la quantité de métal du groupe platine est déposée à raison de 0,3 à 2 pour cent du poids de la zéolite.

12. Procédé selon la revendication 1, dans lequel la quantité du catalyseur est de 0,5 à 30 pour cent du poids du 2,3,5-triméthylbenzoquinone.

13. Procédé selon la revendication 1, dans lequel le solvant est un alcool aliphatique.

14. Procédé selon la revendication 1, dans lequel le solvant est du méthanol ou de l'éthanol.

15. Procédé selon la revendication 1, dans lequel la réduction du 2,3,5-triméthylbenzoquinone par l'hydrogène s'effectue sous une pression partielle d'hydrogène de 0,01 à 20 kg/cm$^2$.

16. Procédé selon la revendication 1, dans lequel la réduction du 2,3,5-triméthylbenzoquinone par l'hydrogène s'effectue à une température allant de 10 à 100°C.

## Patentansprüche

1. In einem Verfahren zur Herstellung von 2,3,5-Trimethylhydrochinon durch Reduzierung von 2,3,5-Trimethylbenzochinon mit Wasserstoff in einem Lösungsmittel in Gegenwart eines Katalysators, die Verbesserung, die den Gebrauch eines Metalls der Platin-Gruppe, aufgebracht auf Zeolith, als Katalysator umfaßt.

2. Verfahren von Anspruch 1, wobei der Zeolith wenigstens ein Material ist ausgewählt aus der Gruppe, die aus Mordeniten, Faujasiten, Chabasiten, einer Molekularsiebart, Klinoptilolith und Erionit besteht.

3. Verfahren nach Anspruch 1, wobei das Metall der Platin-Gruppe wenigstens ein Metall ist ausgewählt aus der Gruppe, die aus Palladium, Platin, Ruthenium und Rhodium besteht.

4. Verfahren nach Anspruch 1, wobei das Metall der Platin-Gruppe Palladium, Platin oder eine Mischung davon ist.

5. Verfahren nach Anspruch 1, wobei das Metall der Platin-Gruppe Palladium ist.

6. Verfahren nach Anspruch 1, wobei der Katalysator ein Material ist erhalten durch Aufbringung einer Platin-Gruppenmetallverbindung auf Zeolith und anschließende Reduzierung der Verbindung.

7. Verfahren nach Anspruch 6, wobei die Aufbringung der Platin-Gruppenmetallverbindungauf Zeolith mit einer Ionenaustauschmethode oder einer Imprägnierungsmethode durchgeführt wird.

8. Verfahren nach Anspruch 6, wobei die Platin-Gruppenmetallverbindung ein Chlorid oder ein Aminnkomplex-Salz ist.

9. Verfahren nach Anspruch 6, wobei die Reduzierung mit Wasserstoff durchgeführt wird.

10. Verfahren nach Anspruch 1, wobei die aufgebrachte Menge des Platin-Gruppenmetalls 0.1 bis 10 Gew.% bezogen auf den Zeolithen ist.

11. Verfahren nach Anspruch 1, wobei die aufgebrachte Menge des Platin-Gruppenmetalls von 0.3 bis 2 Gew.% bezogen auf den Zeolithen ist.

**12.** Verfahren nach Anspruch 1, wobei die Menge des Katalysators von 0.5 bis 30 Gew.%, bezogen auf das 2,3,5-Trimethylbenzochinon ist.

**13.** Verfahren nach Anspruch 1, wobei das Lösungsmittel ein aliphatischer Alkohol ist.

**14.** Verfahren nach Anspruch 1, wobei das Lösungsmittel Methanol oder Ethanol ist.

**15.** Verfahren nach Anspruch 1, wobei die Reduzierung des 2,3,5-Trimethylbenzochinons mit Wasserstoff bei einem Wasserstoffpartialdruck von 0.01 bis 20 kg/cm$^2$ durchgeführt wird.

**16.** Verfahren nach Anspruch 1, wobei die Reduzierung des 2,3,5-Trimethylbenzochinons mit Wasserstoff bei einer Temperatur von 10 bis 100°C durchgeführt wird.